# EUROPÄISCHE PATENTANMELDUNG

(11) **EP 2 439 281 A1**
(43) Veröffentlichungstag der Anmeldung: **11.04.2012**
(21) Anmeldenummer: 10186967.5
(22) Anmeldetag: 08.10.2010
(51) Int. Cl.: C12Q 1/68

(54) **Verfahren zu Authentizitätssicherung von Produkten und Produkt mit einer fälschungssicheren Markierung**

(71) Anmelder: Faber-Castell AG, 90546 Stein (DE)
(72) Erfinder: Nies, Andrea, 67117 Limburgerhof (DE)
(74) Vertreter: Mörtel & Höfner

(57) **Zusammenfassung**

Die Erfindung bezieht sich auf ein Verfahren zur Authentizitätssicherung von Waren, bei dem die Ware mit dem Einzelstrang einer Peptidnukleinsäure, einer Marker-PNA versehen ist.
Die Erfindung betrifft weiterhin ein Produkt, das mit einer eine Peptidnukleinsäure enthaltenden Markierung versehen ist.

## Beschreibung

Fälschungen von Produkten stellen ein erhebliches ökonomisches Problem, insbesondere bei Massenprodukten wie kosmetischen Produkten und Produkten des Schreib-, Mal-, und Zeichenbedarfs dar. Um die Fälschungen zu verhindern, bietet sich eine Markierung der Produkte an. In neuerer Zeit wurde als Markierungssubstanzen Desoxyribonukleinsäuren (DNA) benutzt. Dies hat den Vorteil, dass die DNA, auch wenn sie z.B. nur aus zehn Einheiten (sogenanntes "10-mer") besteht, doch sehr viele Variationsmöglichkeiten (mehr als eine Million) in ihrer Sequenz bietet. Markiert man also mit einem bestimmten 10-mer, so müsste der Fälscher dieses herausfinden und seinem Plagiat hinzufügen.

Aufgabe der Erfindung ist es, ein alternatives Verfahren zur Authentizitätssicherung insbesondere von kosmetischen Produkten aller Art und Schreib-, Zeichen- und Malgeräten sowie von Farben, und ein entsprechendes Produkt mit einer fälschungssicheren Markierung vorzuschlagen.

Diese Aufgabe wird durch ein Verfahren nach Anspruch 1 und ein Produkt nach Anspruch 6 gelöst. Danach wird ein Produkt mit dem Einzelstrang einer Peptidnukleinsäure, einer Marker-PNA, versehen. Unter einem Produkt ist eine Ware in flüssiger oder fester Form mit oder ohne Verpackung oder sonstige Umhüllung zu verstehen. Dabei kann die Ware selbst und/oder ihre Verpackung bzw. Umhüllung mit Marker-PNA versehen werden. PNA wird von Mikroorganismen nicht oder zumindest weniger leicht zersetzt oder verändert als DNA, so dass sie auch nach einer längeren Zeitdauer, etwa im Falle der Markierung langlebiger oder für eine gewisse Zeit gelagerter Produkte, ein Fälschungsnachweis möglich ist. Vorzugsweise wird eine Marker-PNA verwendet, deren Grundgerüst aus 2-Amonoethylglycin-Monomeren aufgebaut ist. PNA ist außerdem chemisch stabiler als DNA, ist beispielsweise unempfindlicher gegenüber Säuren.

Im Zuge einer Fälschungsüberprüfung wird die Marker-PNA entweder direkt am Produkt oder nach Entnahme beispielsweise durch eine Extraktion mit einem komplementären Einzelstrang einer Nukleinsäure, einer Sonden-NA, in Kontakt gebracht wird. Im Falle einer weitgehenden Übereinstimmung zwischen Marker-PNA und Sonden-NA kommt es zu einer Hybridisierung, d.h. es bilden sich Doppelstränge aus den Hybridisierungspartnern. Um eine Hybridisierung nachzuweisen, ist die Sonden-NA mit einem detektierbaren Label versehen. Unter einem Label ist hier beispielsweise ein Molekül, ein Molekülbestandteil oder ein Partikel zu verstehen, wobei sich ein solches Label aufgrund einer Fluoreszenz oder einer magnetischen Eigenschaft mit Hilfe einer entsprechenden Einrichtung detektieren lässt. Vorzugsweise wird dabei so vorgegangen, dass die Marker-PNA oder die Sonden-NA auf einer Oberfläche immobilisiert ist, und der jeweils mobile Hybridisierungspartner das detektierbare Label trägt.

Die Sonden-NA ist eine e oder PNA, wobei letzter bevorzugt ist.

Das der DNA strukturell verwandte, in der untenstehenden Abbildung gezeigte PNA-Molekül weist ein Pseudopeptidgerüst auf, von dem in regelmäßigen Abständen kurze Seitenketten abzweigen, welche eine der vier Nukleobasen Adenin (A), Thymin (T), Cytosin (C) und Guanin (G) tragen.

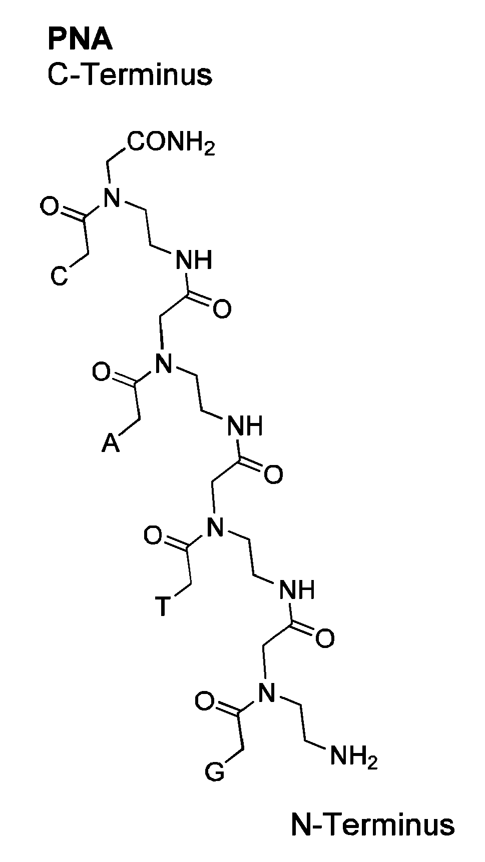

Aus der Struktur der PNAs ergibt sich, dass sie durch ihre Basensequenz charakterisiert sind. Diese Sequenzen sind wie DNA in der Lage, mit komplementären Sequenzen zu hybridisieren. Die Markierung eines Produkts mit einer PNA kann letztlich auf beliebige Art und Weise erfolgen. Es kann dem eigentlichen Produkt, beispielweise der kosmetischen Zubereitung eines kosmetischen Produkts, etwa der Minenmasse eines Stiftes, einer Creme, einem Puder o.dgl. beigemengt werden. Alternativ kann auch die jeweilige Verpackung oder Umhüllung, etwa eine Stifthülle, ein Behälter mit PNA markiert werden. Dies kann z.B. dadurch erfolgen, dass die Marker-PNA in einer auf der Oberfläche des Behälters vorhanden Beschichtungsmasse eingebracht ist. Sie können den jeweiligen Materialien in geringer Menge zugesetzt werden und werden, falls erforderlich, wieder aus jenen rückisoliert und durch Hybridisierung auf oberflächenbasierten Nachweissystemen detektiert.

### Beispiele:

### Dotierung von Leinöl mit Marker-PNA der Sequenz ttt ttt ttt tgt aac tgt tca tcc c

Die zehn Thymine der PNA dienen zum Nachweis im späteren Dreistrangsystem (s.u.) 1 ml Leinöl wird mit 40 ng PNA (gelöst in 50 µl Ethanol/Wasser (9/1)) versetzt und anschließend 5 min durch maschinelles Schütteln homogenisiert.

### Dotierung von Wasserlack mit Marker-PNA der Sequenz ttt ttt ttt tcg cat acg tcc tac g

Die zehn Thymine der PNA dienen zum Nachweis im späteren Dreistrangsystem (s.u.) 200 µg PNA werden in 200 µl Wasser gelöst und in 20 g (etwa 20 ml) Wasserlack homogen durch Rühren verteilt.

### Extraktion der Marker-PNA

### 1. LEINÖL

Die Leinölprobe wird mit 1 ml Hexan verdünnt und im Anschluss mit 200 µl 0,1 M Salzsäure ausgeschüttelt. Nach Zentrifugation wird die Wasserphase abgetrennt und zur Trockene gebracht. Der Rückstand wird in 50 µl Hybridisierungspuffer aufgenommen.

### 2. WASSERLACK

Der Lack wird von einem Bleistift mittels Skalpell entfernt und in 100 µl Trifluoressigsäure/Tetrahyrdofuran (1/1) solubilisiert. Nach fünfminütigem Schütteln wird das Volumen stark vermindert (Vakuumzentrifuge), nicht aber zur Trockene gebracht. Es werden danach 200 µl Chloroform zugesetzt und eine Minute maschinell geschüttelt. Anschließend gibt man 400 µl 1M Trifluoressigsäure hinzu und schüttelt für 30 Minuten. Nach fünfminütigem Zentrifugieren wird die klare, obere, wässrige Phase abgenommen und zur Trockene gebracht. Der Rückstand wird in 50 µl PBS-Puffer (pH=7,4) aufgenommen.

### Detektion der Dotierungen

Microarray:
Glasobjektträger mit PEGMA-Beschichtung (Polyethylenglykolmethacrylat) mit NH₂-Gruppen, Aktivierung mittels Di-(N,N'-succinimidyl)-carbonat, Umsetzung der Glasoberfläche mit zur detektierenden PNA komplementären PNA. Die Glasoberfläche trägt jetzt kovalent angebundene Sonden-PNA.

Hybridisierung:
Die eben beschriebene Glasoberfläche wird mit den 50 µl PBS-Puffer, die vom Leinöl bzw. Wasserlack stammen, bedeckt und 12 Stunden so belassen. Dadurch kommt es zu einer Hybridisierung zwischen oberflächengebundener Sonden-PNA und Marker-PNA. Letztere trägt, am vom Chip entfernten Ende, noch zehn freie Thyminreste. Nach Waschen mit PBS-Puffer (pH=7,4) erfolgt die Zugabe eines detektierbaren Labels in Form einer Cy5-markierten DNA-15-mer, welches nur aus Adeninresten aufgebaut ist. Ein Teil der Adeninreste hybridisiert mit den freien Thyminresten der Marker-PNA. Nach Waschen in PBS-Puffer (pH=7,4) und Trocknen im Stickstoffstrom wird die Fluoreszenz der Spots mit einem üblichen Fluoreszenzscanner ausgelesen.

## Patentansprüche

1. Verfahren zur Authentizitätssicherung von Waren, bei dem die Ware mit dem Einzelstrang einer Peptidnukleinsäure, einer Marker-PNA versehen ist.

2. Verfahren nach Anspruch 2,
**gekennzeichnet durch**
die Verwendung einer Marker-PNA, deren Grundgerüst aus 2-Amonoethylglycin-Monomeren aufgebaut ist.

3. Verfahren nach Anspruch 1 oder 2,
**dadurch gekennzeichnet,**
**dass** die Marker-PNA der Ware mit einem komplementären Einzelstrang einer Nukleinsäure, einer Sonden-NA in Kontakt gebracht wird und Hybridisierungsereignisse, d.h. die Bildung von Doppelsträngen aus Marker-PNA und Sonden-NA, mit Hilfe eines an die Marker-PNA oder die Sonden-NA gebundenen detektierbaren Labels erfasst werden.

4. Verfahren nach Anspruch 3,
**dadurch gekennzeichnet,**
**dass** die Marker-P NA oder die Sonden-NA auf einer Oberfläche immobilisiert ist, und der jeweils mobile Hybridisierungspartner das detektierbare Label trägt.

5. Verfahren nach einem der vorhergehenden Ansprüche,
**dadurch gekennzeichnet,**
**dass** als Sonden-NA eine Peptidnukleinsäure verwendet wird.

6. Produkt,
**dadurch gekennzeichnet,**
**dass** es eine Markierung aufweist, die eine Peptidnukleinsäure enthält.

7. Produkt nach Anspruch 6,
**dadurch gekennzeichnet,**
**dass** es sich um ein kosmetisches Produkt handelt.
